# EUROPEAN PATENT APPLICATION

(11) **EP 0 539 223 A1**
(43) Date of publication of application: **28.04.1993**
(21) Application number: 92309738.0
(22) Date of filing: 23.10.1992
(51) Int. Cl.: C07C 311/29, A61K 31/18

(54) **Glycine derivative monosodium salt tetrahydrate having an inhibitory effect on elastase**

(30) Priority: 25.10.1991 JP 306925/91
(71) Applicant: ONO PHARMACEUTICAL CO., LTD., Chuo-ku Osaka 541 (JP)
(72) Inventor: Imaki, Katsuhiro, Minase Research Institute, Mishima-gun, Osaka (JP); Wakatsuka, Hirohisa, Minase Research Institute, Mishima-gun, Osaka (JP)
(74) Representative: Bentham, Stephen

(57) **Abstract**

N-[o-(p-pivaloyloxybenzenesulfonylamino)benzoyl] glycine monosodium salt tetrahydrate of the formula (I) : 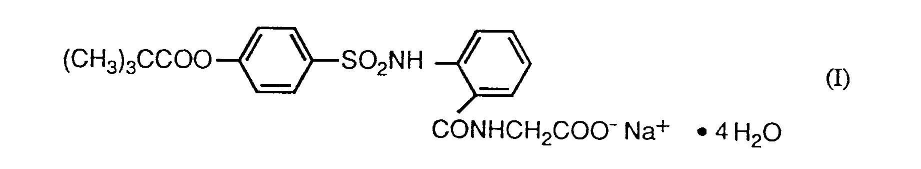
is a non-hygroscopic, homogeneous and stable compound, and has an inhibitory effect on elastase.

## Description

The present invention relates to a glycine derivative, in particular to N-[o-(p-pivaloyloxybenzenesulfony- lamino)benzoyl] glycine monosodium salt tetrahydrate, a process for its preparation, pharmaceutical compositions containing it and to a process for the preparation of N-[o-(p-pivaloyloxybenzenesulfonyl amino)benzoyl] glycine as an intermediate in its preparation.

Lysosomal hydrolases of neutrophils have an important role in defense reactions against tissue damage caused by microbes or inflammation.

Elastase and cathepsin G, which are neutral serine proteinases exist locally in azurophil granules and play a part in decomposition of connective tissue.

In particular, elastase degrades elastic connective tissue by cleaving the cross-linking of elastin which directly maintains the elasticity of e.g. lung tissue by cleaving the hydrophobic part of protein [J. Cell. Biol., 40, 366 (1969)] and selectively degrading the cross-linking area of collagen as well as elastin [J. Biochem., 84, 559 (1978)]. It also acts on tissue proteins such as proteoglycans [J. Clin. Invest., 57, 615 (1976)]. It will be seen, therefore, that elastase plays an important role in metabolism of connective tissue.

Elastase is inactivated by al-proteinase inhibitor (u₁,-PI) which is a common inhibitor for serine, proteinases in vivo and an imbalance of enzyme and inhibitor causes the destruction of tissue [Schweiz. Med. Wshr., 114, 895 (1984)].

The turnover of elastin in normal tissue is very slow [Endocrinology, 120, 92 (1978)], but pathological acceleration in degradation of elastin is found in various disease conditions such as pulmonary emphysema [Am. Rev. Respir. Dis., 110, 254 (1974)], atherosclerosis [Lab. Invest., 22, 228 (1970)] and rheumatoid arthritis [in Neutral Proteases of Human Polymorphonuclear Leukocytes, Urban and Schwarzenberg, Baltimore - Munich (1978), page 390], which suggests a relationship between elastase and diseases [Infection Inflammation Immunity, g, 13 (1983)].

In view of this many studies on the development of elastase inhibitors have been conducted, various substances inhibiting elastase have been proposed and many patent applications have been filed.

For example, in the specification of European Patent Publication No. 347168, it is disclosed that compounds of the formula (A): 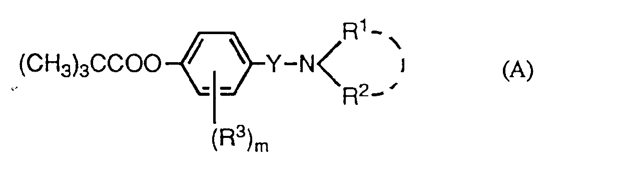
wherein Y represents a sulfonyl or carbonyl group, (1) R¹ and R², which may be the same or different, each represent
a hydrogen atom;
an alkyl group of up to 16 carbon atom(s) optionally substituted by a carboxy group;
a group of the formula: 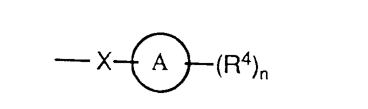
   wherein X represents a single bond, a sulfonyl group, an alkylene group of up to 4 carbon atom(s) or an alkylene group of up to 4 carbon atom(s) substituted by a carboxy group or benzyloxycarbonyl group, 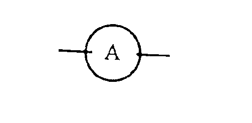
   represents a carbocyclic ring or heterocyclic ring,
   n represents an integer of 1 to 5,
   the groups R⁴ independently represent,
   (a) hydrogen atom or an alkyl group of up to 8 carbon atom(s),
   (b) an alkoxy group of up to 14 carbon atom(s),
   (c) an alkylthio group of up to 6 carbon atom(s),
   (d) a hydroxy group, halogen atom, nitro group or trihalomethyl group,
   (e) a group of the formula: -NR^{4l}R⁴²
      wherein R⁴¹ and R⁴² each represents,
      independently, a hydrogen atom or an alkyl group of up to 4 carbon atom(s),
   (f) a tetrazole group,
   (g) a sulfonic acid group or hydroxymethyl group,
   (h) a group of the formula: -SO₂NR⁴¹R⁴²
      wherein R⁴¹ and R⁴² are as hereinbefore defined,
   (i) a group of the formula: -Z⁴¹-COOR⁴³
      wherein Z⁴¹ represents a single bond, an alkylene group of up to 4 carbon atom(s) or an alkenylene group of from 2 to 4 carbon atoms,
      and R⁴³ represents a hydrogen atom, an alkyl group of up to 4 carbon atom(s) or a benzyl group,
   (j) a group of the formula: -CONR⁴¹R⁴²
      wherein R⁴¹ and R⁴² are as hereinbefore defined,
   (k) a group of the formula: -COO-Z⁴²⁻COOR⁴³° wherein Z⁴² represents an alkylene group of up to 4 carbon atom(s), and
      R⁴³⁰ represents a hydrogen atom or an alkyl group of up to 4 carbon atom(s),
   (I) a group of the formula: -COO-Z⁴²⁻CONR41R42
      wherein Z⁴², R⁴¹ and R⁴² are as hereinbefore defined,
   (m) a group of the formula: -OCO-R⁴⁵
      wherein R⁴⁵ represents an alkyl group of up to 8 carbon atom(s) or a p-guanidinophenyl group,
   (n) a group of the formula: -CO-R⁴⁶
      wherein R⁴⁶ represents an alkyl group of up to 4 carbon atom(s),
   (o) a group of the formula: -O-Z⁴³⁻COOR⁴⁵⁰
      wherein Z⁴³ represents an alkylene group of up to 6 carbon atom(s), and R⁴⁵⁰ represents a hydrogen atom, an alkyl group of up to 8 carbon atom(s) or a p-guanidinophenyl group,
   (p) a group of the formula: 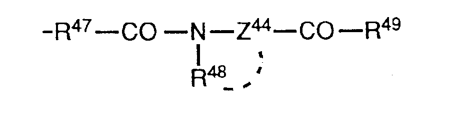
      wherein >N-Z⁴⁴⁻CO represents an amino acid residue, R⁴⁷ represents a single bond or an alkyl group of up to 4 carbon atom(s), R⁴⁸ represents a hydrogen atom or an alkyl group of up to 4 carbon atom(s), and R⁴⁹ represents a hydroxy group, alkoxy group of up to 4 carbon atom(s), amino group, amino group substituted by one or two alkyl groups of up to 4 carbon atom(s), carbamoylmethoxy or carbamoylmethoxy group substituted by one or two alkyl group of up to4 carbon atom(s) at the nitrogen atom of the carbamoyl group, or wherein 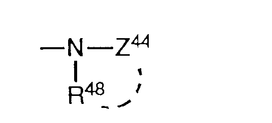
   represents a heterocyclic ring containing 3 to 6 carbon atoms and R⁴⁷ and R⁴⁹ has each same meaning as described hereinbefore, or
      (2) R¹, R² and the nitrogen atom bonded to R¹ and R² together represent a heterocyclic ring containing at least one nitrogen atom(s) and substituted by -COOH, or an unsubstituted heterocyclic ring containing at least one nitrogen atom(s),
      R3 represents
      (1) a hydrogen atom,
      (2) a hydroxy group,
      (3) an alkyl group of up to 6 carbon atom(s),
      (4) a halogen atom,
      (5) an alkoxy group of up to 4 carbon atom(s) or
      (6) an acyloxy group of 2 to 5 carbon atoms,

      and m represents an integer of up to 4,

and pharmaceutically acceptable salts thereof, which have an inhibitory effect on elastase and are, therefore, useful for the treatment of diseases induced by elastase, e.g. pulmonary emphysema, atheroscleorosis or rheumatoid arthritis.

The compound of formula (A), wherein Y is a sulfonyl group, one of R¹ and R² is a hydrogen atom, and the other is a group of the formula: 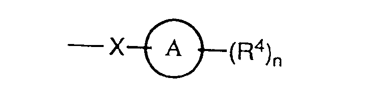
in which X is single bond, 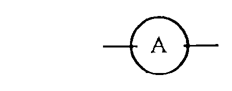
is a benzene ring, n is 1, and R⁴ is a group of the formula: 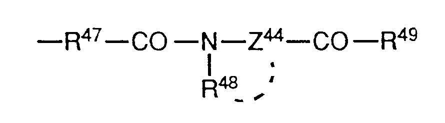
in which >N-Z44-CO is an amino acid residue (glycine), R⁴⁷ is a single bond, R⁴⁸ is a hydrogen atom and R⁴⁹ is a hydroxy group, is specifically disclosed in Example 2 (63) in the specification of European Patent Publication No. 347168.

The known compound was found to have unsatisfactory water solubility. In the course of investigations to discover a means of improving the solubility various non-toxic salts of the compound of the formula (II) were prepared. As a result of the investigations, it was concluded that a sodium salt was the most suitable. However, the monosodium salt of the compound of the formula (II) is hygroscopic and is hydrated with the passage of time on exposure to air, finally to form a tetrahydrate via a mixture of the monohydrate, dihydrate and trihydrate.

Hygroscopicity is undesirable in a pharmaceutical whose physiochemical characteristics should remain stable to permit the reliable formulation of pharmaceutical compositions.

It has been found that if the monosodium salt of the compound of the formula (II) is synthesized as its tetrahydrate the compound is stable at ambient temperatures and relative humidities and neither gains nor loses water as ambient temperatures and relative humidities vary.

There is no description of the tetrahydrate itself and no suggestion thereof in the specification of European Patent Publication No. 347168. Nor would it be expected from that specification that the monosodium salt would prove to be hygroscopic or that such hygroscopic properties could be removed by forming the tetrahydrate thereof.

The present invention accordingly provides N-[o-(p-pivaloyloxybenzenesulfonylamino)benzoyl] glycine monosodium salt tetrahydrate of the formula (I):

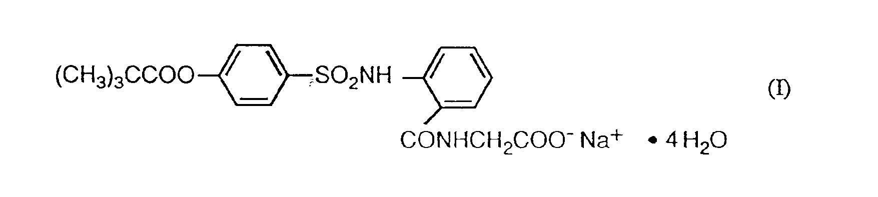

The invention also provides a process for the preparation of the compound of formula (I) which comprises reacting a compound of the formula (II): 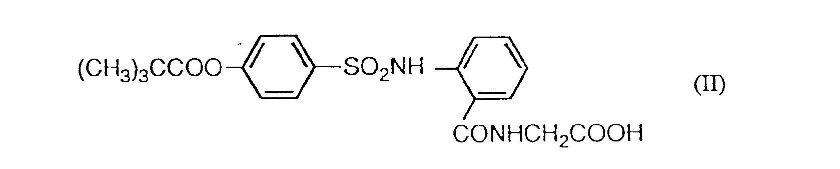
with an aqueous solution of sodium hydroxide, sodium bicarbonate or sodium carbonate (preferably 1.0 to 1.2 molecular equivalents thereof) in an inert organic solvent (e.g., tetrahydrofuran (THF), dioxan or acetone), recrystallizing the product obtained (generally crude crystals after removal of the solvent) from water or a mixture of a water miscible organic solvent and water (e.g. aqueous methanol, aqueous ethanol, aqueous propyl alcohol, aqueous acetone or aqueous dioxane), and then air-drying the obtained crystals, preferably for more than 24 hours.

The present invention also provides a novel process for the preparation of the compound of the formula (II).

European Patent Publication No. 347168 filed by the present applicant, describes the preparation of the compound of formula (II) by a series of reactions depicted in the following scheme [A] (only that part relating to the compound of the formula (II) is depicted).

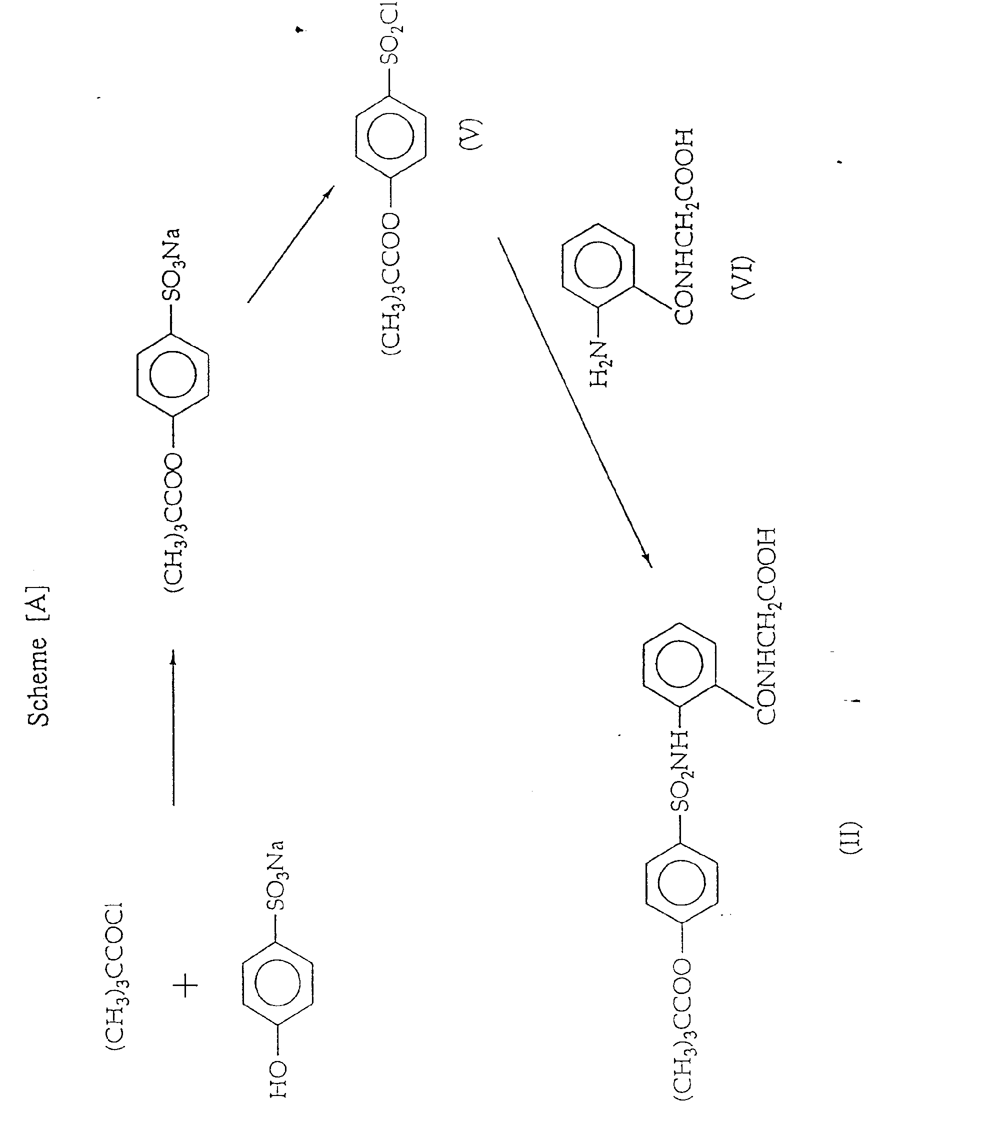

However, the following problem exists in the method described above for the preparation of the compound of the formula (II) on an industrial scale. The compound of the formula (V) may react not only with an amino group but also partially with the carboxyl group in the compound of formula (VI) in the amidation in scheme [A] because of the fairly weak nucleophilicity of the amino group in the compound of the formula (VI). Mixed acid anhydrides may be prepared by this reaction and various by-products may be formed by subsequent reactions. This makes it very difficult to improve the yield of the compound of the formula (II) (the yield of the compound of the formula (II) from that of the formula (V) is about 30%).

Accordingly, the method disclosed in the above specification is not entirely satisfactory as a method for the industrial preparation of the compound of the formula (II).

As a result of investigations into the preparation of the compound of the formula (II), it has been found that the compound can be industrially advantageously prepared by using the series of reactions depicted in the following scheme [B].

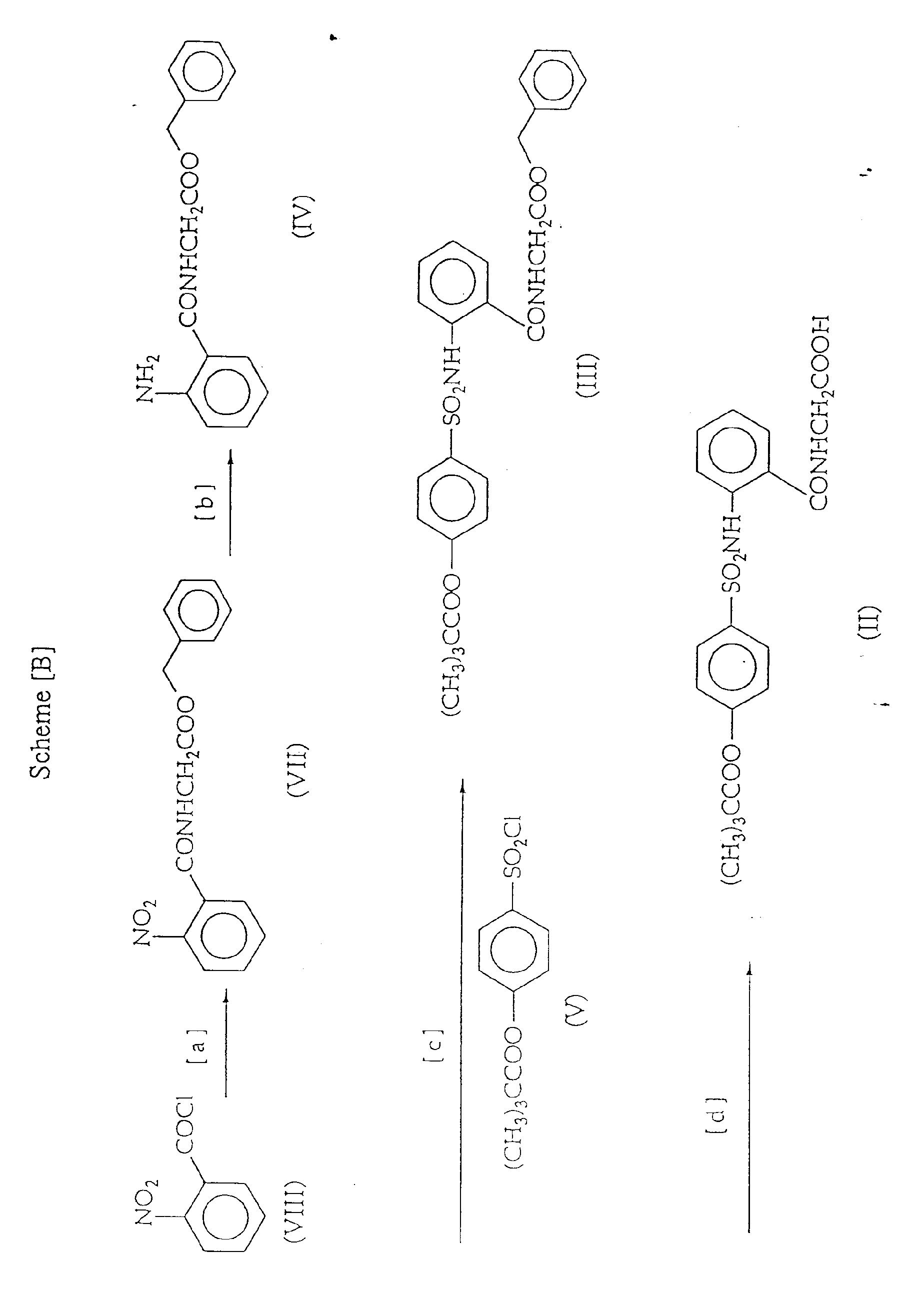

In scheme [B], the use of the compound of the formula (IV) in which the carboxyl group is protected by a benzyl group, in the sulfonamidation reaction (step [c]) reduces the formation of by-products, as compared with the conventional method in scheme [A] where the compound of the formula (VI) is used. Further, the compound of the formula (III) prepared by the sulfonamidation reaction can be easily purified and can be quantitatively reduced to obtain the compound of formula (II) in high purity and yield (the yield of the compound of the formula (II) from that of the formula (IV) is about 85%).

Each step in scheme [B] is described below:
Step [a], which is the amidation, may be carried out by reacting an acyl halide with the amine of the formula (IX): 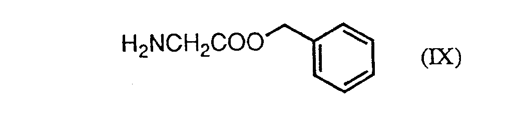
   in the presence of an amine (e.g. triethylamine, pyridine or picoline), in an inert solvent [e.g. a halogenated hydrocarbon (such as methylene chloride, chloroform, carbon tetrachloride, tetrachloroethane or hexachloroethane), an ether (such as tetrahydrofuran (THF), tetrahydropyran, dioxan, diethyl ether, dimethyl ether, diisopropyl ether, diphenyl etherorethyl methyl ether) or benzene analogue (e.g. benzene, toluene orxylene)], at a temperature of -20~ 50°C.
Step [b], which is the reduction, may be carried out by reacting the nitro compound in the presence or absence of an inert solvent [e.g. an ether as hereinbefore described, an alcohol (e.g. methanol, ethanol or propanol), water or a mixture thereof], using iron, zinc or tin and an acid (e.g. concentrated hydrochloric acid, concentrated sulfuric acid or acetic acid at from room temperature to the reflux temperature of the solvent, or by reaction in the presence of an alcohol (as hereinbefore described), using sodium borohydride (NaBH₄) and nickel chloride (NiC1₂), at a temperature of-20~50°C, or a system of sodium sulfide / ammonium chloride / aqueous solution of ammonia, by reaction in the presence or absence of an inert solvent [e.g. an alcohol or an ether (as hereinbefore described), or using a system of sodium hydrosulfite (Na₂S₂0₄) / aqueous solution of ammonia in the presence or absence of an inert solvent [e.g. an alcohol or an ether (as hereinbefore described)], or by reaction in a mixture of N,N-dimethylformamide (DMF) or 1, 3-dimethyl-2-imidazolinone (DMI), and acetic acid, in the presence of raney nickel at normal or elevated pressure of hydrogen gas, at a temperature of 30~100°C, preferably 35-40°C.
Step [c], which is the sulfonamidation, may be carried out by reacting a sulfonyl chloride of the formula (V) with a compound of the formula (IV), in the presence of an amine (as hereinbefore described) in an inert solvent [e.g. a halogenated hydrocarbon (as hereinbefore described) or an ether (as hereinbefore described) or without a solvent, at a temperature of -20~50°C.

The present invention provides, in particular, a process for the preparation of N-[o-(p-pivaloyloxybenze- nesufonylamino)benzoyl]glycine of the formula (II) as an intermediate for the preparation of a compound of the formula (I), which process comprises reducing a compound of the formula (III):

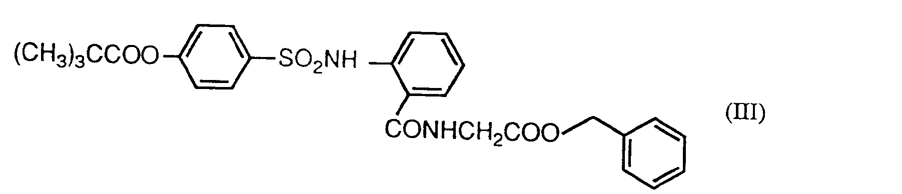

Step [d], which is the reduction, may be carried out by reacting a compound of the formula (III) in an inert solvent [e.g. a halogenated hydrocarbon, an ether or an alcohol (as hereinbefore described), DMF, acetic acid, ethyl acetate, or a mixture of two or more of them] in the presence of a hydrogenation catalyst (e.g. palladium on carbon, palladium black, palladium, platinum dioxide, nickel or raney nickel) at normal or elevated pressure of hydrogen gas, at a temperature of 0-200°C, preferably at room temperature.

The starting materials and reagents in the process of the present invention are known per se, or may be prepared by known methods.

For example, the compound of the formula (VIII) is on the market, and the process for the preparation of the compound of the formula (V) is described in the specification of the European Patent Publication No. a 347168.

N-[o-(p-pivaloyloxybenzenesulfonylamino)benzoyl] glycine monosodium salt tetrahydrate, of the formula (I) of the present invention is a non-hygroscopic, homogeneous and stable compound, and has an inhibitory effect on elastase. Accordingly, the compound may be useful for the treatment and/or prevention of diseases induced by the abnormal enhancement of degradation of elastin, collagen fibre and/or proteoglican by the action of elastase (e.g. pulmonary emphysema, atherosclerosis or rheumatoid arthritis) in mammals, especially in human beings.

The hygroscopicity and pharmacological effect of the compound of the present invention have been confirmed by various experiments.

### 1. Hygroscopicity

Method:
N-[o-(p-pivaloyloxybenzenesulfonylamino)benzoyl] glycine monosodium salt tetrahydrate (the compound of the present invention) and N-[o-(p-pivaloyloxybenzenesulfonylamino)benzoyl] glycine monosodium salt (the comparison compound) were left at a temperature of 25°C and a relative humidity of 75%. The hygroscopicity of each compound was measured by the ratio of its weight to the starting weight with the passage of time. The results are shown in the following Table 1.

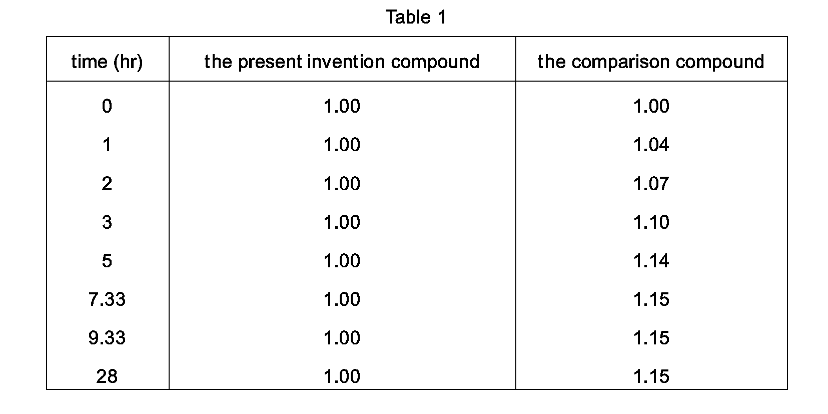

The compound of the present invention showed no change of its weight, though the comparison compound increased its weight with time. Accordingly, it can be seen that the compound of the invention is a non-hygroscopic, homogeneous and stable compound.

### 2. Inhibitory effect on elastase

The test was carried out by slight modification of the method of Bieth et al. [see Biochem. Med., 75, 350 (1974)] using elastase from human neutrophil. It is a spectrophotometric method using the synthesized substrate [succinyl-alanyl-prolyl-alanyl-p-nitroanilide (Suc-Ala-Pro-Ala-pNA, produced by peptide laboratory)] which has comparatively high specificity on neutrophil elastase.

The reaction mixture consisted of 1mM Suc-Ala-Pro-Ala-pNA (dissolving in N-methylpyrrolidone to the concentration of 100mM, and then adding 1/100 amount of the solution to the reaction mixture), 0.1M Tris-HCI buffer (pH 8.0), 0.2M sodium chloride aqueous solution, the sample solution of various concentrations and enzyme solution in a final volumme of 1.0 ml was incubated at 37°C for 30 minutes. The reaction was stopped by the addition of 100µl of 50% acetic acid into the reaction mixture, and then p-nitroanilide released was measured by the absorbance at405 nm. Inhibition percentage and IC₅₀ value, of the test compound were calculated.

The result showed that the IC₅₀ value of the compound of the present invention, of the formula (I), was 0.046_{w}M.

The toxicity of the compound of the present invention was very low, so that they may be considered to be safe for pharmaceutical use.

The compound of formula (I) has an inhibitory effect on elastase. Accordingly, it is useful for the treatment and/or prevention of diseases induced by the abnormal enhancement of the degradation of elastin, collagen fiber and/or proteoglican, by the action of elastase (e.g. pulmonary emphysema, atherosclerosis or rheumatoid arthritis).

For the purpose described above, the compound of formula (I) will normally be administered systemically or partially, usually by oral or parenteral administration.

The dose to be administered is determined depending upon age, body weight, symptom, desired therapeutic effect, route of administration, and the duration of the treatment. In the human adult, the doses per person per dose are generally from 50mg to 500mg, by oral administration, up to several times per day, and from 10mg to 200mg, by parenteral administration up to several times per day, or continuous administration between 1 and 24 hrs. per day intravenously.

As mentioned above, the doses to be used depend upon various conditions. Therefore, there are cases in which doses lower than or greater than the ranges specified above may be used.

The compounds of the preent invention may be used as, for example, solid compositions, liquid compositions or other compositions for oral administration, or as injections, liniments or suppositories for parenteral administration.

Solid compositions for oral administration include compressed tablets, pills, capsules, dispersible powders, and granules. Capsules include hard capsules and soft capsules.

In such solid compositions, the active compound is admixed with at least one inert diluent (e.g. lactose, mannitol, glucose, hydroxypropyl celluose, microcrystalline cellulose, starch, polyvinylpyrrolidone or magnesium metasilicate aluminate). The compositions may also comprise, as is normal practice, additional substances other than inert diluents, e.g. lubricating agents such as magnesium stearate, disintegrating agents such as cellulose calcium glycolate, stabilizing agents such as lactose and solubilisers such as glutamic acid or asparaginic acid.

The tablets or pills may, if desired, be coated with a film of gastric or enteric material (e.g. sugar, gelatin, hydroxypropyl cellulose or hydroxypropylmethyl cellulose phthalate); two or more layers may be used. Compositions for oral administration also include capsules of absorbable materials such as gelatin.

Liquid compositions for oral administation include pharmaceutically-acceptable solutions, emulsions, suspensions, syrups and elixirs containing inert diluent(s) commonly used in the art such as purified water or ethanol.

Besides inert diluents, such compositions may also comprise adjuvants such as wetting agents and, suspending agents, sweetening agents, flavouring agents, perfuming agents and preserving agents.

Other compositions for oral administration include spray compositions which may be prepared by known methods and which comprise the active compound.

Spray compositions may comprise additional substances other than inert diluents, e.g. stabilizing agents, such as sodium sulfite, and isotonic buffers such as sodium chloride, sodium citrate or citric acid.

Forthe preparation of such spray compositions, for example, the method described in United States Patent No. 2,868,691 or 3,095,355 may be used.

Preparations for parenteral administration by injection include sterile aqueous or non-aqueous solutions, suspensions and emulsions. In such compositions, the active compound is admixed with at least one inert aqueous diluent (e.g. distilled water for injection or physiological salt solution) or inert non-aqueous diluent (e.g. propylene glycol, polyethylene glycol, olive oil, ethanol or POLYSORBATE 80 (registered trade mark)). These compositions may also comprise adjuvants other than inert diluents, e.g. preserving agents, wetting agents, emulsifying agents, dispersing agents, stabilizing agents (e.g. lactose, tonicity agents, buffering agents, and solubilisers (e.g. glutamic acid or asparaginic acid).

They may be sterilized forexample, by filtration through a bacteria-retaining filter, by incorporation of sterilizing agents in the compositions or by irradiation. They may also be manufactured in the form of sterile solid compositions, for example, by freeze-drying, which can be dissolved in sterile water or some other sterile diluent for injection immediately before use.

Other compositions for parenteral administration include liquids for external use, and endermic liniments (e.g. ointments), suppositories and pessaries which comprise the active compound and may be prepared by known methods.

The followng Reference Examples and Examples illustrate the present invention.

The solvents in the parentheses show the developing or eluting solvents and the ratios of the solvents used are by volume in thin layer chromatographic (TLC) separation. Unless otherwise specified, infrared absorption spectrum (IR) was measured by KBr method.

### Reference Example 1

N-(2-nitrobenzoyl)glycine benzyl ester

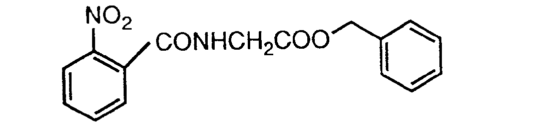

Triethylamine (4.6ml) was added to a mixture of glycine benzyl ester p-toluene sulfonic acid salt (4.049g) and methylene chloride (20ml). To the mixture thus obtained, 2-nitrobenzoyl chloride (1.322ml) was added dropwise under cooling with ice, and the mixture was stirred for one hour at room temperature.

The reaction solution was acidified by adding 1 N hydrochloric acid, and the organic layer was washed with water, an aqueous solution of sodium bicarbonate, water and a saturated aqueous solution of sodium chloride, successively, dried over magnesium sulfate and concentrated under reduced pressure to give the title compound (3.16g) having the following physical data.

TLC:Rf 0.7 (chloroform: methanol: acetic acid= 30:3:1).

### Reference Example 2

N-(2-aminobenzoyl) glycine benzyl ester

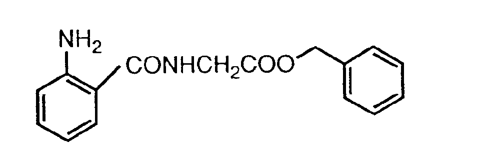

Powder of iron (3.02g) was added by portions to a solution of the nitro compound (2.82g) obtained in Reference Example 1 in a mixture of THF (18ml) and water (9ml) at room temperature.

2N hydrochloric acid (1.08ml) was added dropwise to the mixture, and the mixture was stirred overnight at room temperature. 2N aqueous solution of sodium hydroxide (1.2mi) was added to the obtained reaction solution, and the mixture was filtered through celite and extracted with ethyl acetate. The extract was washed with water and a saturated aqueous solution of sodium chloride, successively, dried over magnesium sulfate and concentrated under reduced pressure to give the title compound (2.05g) having the following physical data.

TLC:Rf 0.6 (chloroform: methanol: acetic acid = 100:5:1).

### Reference Example 3

N-(2-aminobenzoyl) glycine benzyl ester (another process for the preparation)

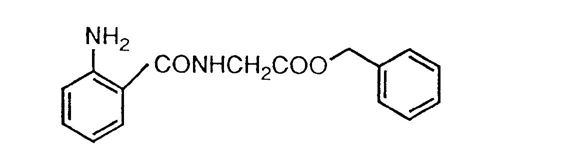

Raney nickel (8.4g) was added by portions to 5N aqueous solution of sodium hydroxide (40ml) at 50°C over 30 minutes.

After the mixture was stirred for one hour at 50°C, raney nickel was washed with water. Then the water was displaced by 1,3-dimethyl-2-imidazolinone (DMI).

The mixture of raney nickel (4.2g), the nitro compound (14g) obtaned in Reference Example 1, acetic acid (1.4ml) and DMI (70ml) was stirred for three hours at 40°C under 25 atmospheric pressure of hydrogen in an autoclave.

The reaction solution was filtered through celite and the filtrate was poured into water. The resulting precipitate was collected by filtration. The solid was air-dried at room temperature for a week to give the title compound (11.2g) having the following physical data.

TLC:Rf 0.6 (chloroform: methanol: acetic acid = 100:5:1).

### Example 1

N-[o-(p-pivaloyloxybenzenesulfonylamino)benzoyl] glycine benzyl ester

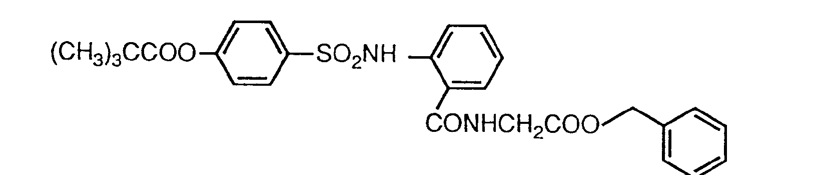

p-pivaloyloxybenzenesulfonyl chloride (prepared by the methods described in Reference Example 5 in the specification of the European Patent Publication No. 347168; 2.66g) was added to a mixture of the amine compound (2.28g) obtained in Reference Example 2 or 3 and pyridine (36ml) under cooling with ice and the mixture was stirred overnight at room temperature.

The reaction solution was added to a mixture of concentrated sulfuric acid and water (52ml/100ml) and extracted with ethyl acetate. The extract was washed with water, an aqueous solution of sodium bicarbonate, water and a saturated aqueous solution of sodium chloride, successfully, dried over magnesium sulfate and concentrated under reduced pressure.

The residue was crystallized from n-hexane, and the crystals were filtered and dried under reduced pressure to give the title compound (3.70g) as pale yellow powder having the following physical data. TLC:Rf 0.64 (ethyl acetate : n-hexane = 1:1);
IR(cm⁻¹): v 3425,2976,1757,1642,1597,1540,1495,1457,1406;
NMR (CDCI₃) 8 (ppm):7.75~7.65(3H,m)7.50~7.30(6H,m),7.18~7.00(3H,m),6.41(1H,brs),5.21(2H,s),4.04(2H,d), 1.30(9H,s).

### Example 2

N-[o-(p-pivaloyloxybenzenesulfonylamino) benzoyl] glycine

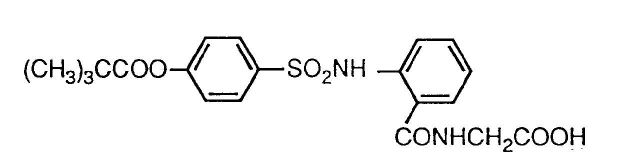

The mixture of the compound obtained in Example 1 (3.70g), methanol (40ml) and 10% palladium on carbon (390mg) was stirred for three hours under an atmospheric pressure of hydrogen at room temperature.

The reaction solution was filtered through celite and concentrated under reduced pressure to give the title compound (2.91g) as white powder having the following physical data.
TLC:Rf 0.14 (chloroform: methanol: acetic acid = 100:5:1);
IR (cm-¹) : v 3432,2978,1749,1722,1647,1524,1493,1453,1407,
NMR(CDCI₃+CD₃0D) δ (ppm) : 7.77(2H,d,J=8.5Hz),7.63(1 H,d J=7.5Hz),7.63(1H,d,J=7.5Hz),7.46(1Ht,J=7.5Hz), 7.15(2H,d, J=8.5Hz),7.13(1H,t,J=7.5Hz),3.98(2H,s),1.35(9H,s).

### Example 3

N-[o(p-pivaloyloxybenzenesulfonylamino)benzoyl] glycine monosodium salt tetrahydrate

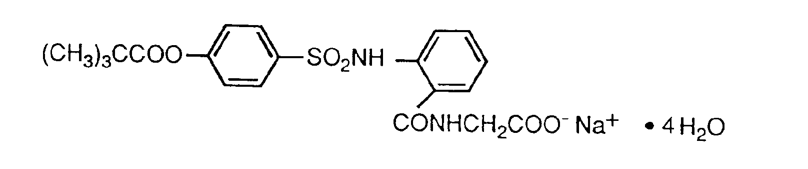

The compound obtained in Example 2 (8.69g) was dissolved with heating into THF (40ml). 5N aqueous solution of sodium hydroxide (4.2ml) was added to the solution under cooling, and the solvent was distilled off under reduced pressure.

The obtained crude crystals were dissolved with heating into water (30ml) and left at 5~10°C overnight. The precipitated crystals were collected by filtration, washed with ice-water and air-dried for 24 hours at room temperature to give the title compound (8.55g) as white powder having the following physical data.

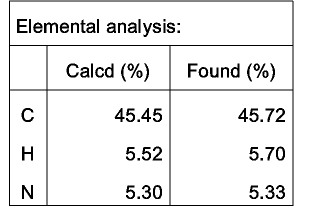

Moisture content (Karl Fischer's method): 13.65% (calc.13.63%)
IR(cm-1): v 3440,2979,1755,1626,1589,1494,1401,1344,1273 1217,1155,1126,1094,942,848,754,687,600,576; The molecular structure based on X-ray diffraction of the tetrahydrate is shown in Figure I.

### Formulation example

The following components were admixed in conventional manner. The solution was sterilized in conventional manner, 5ml portions were placed into vials and feeze-dried to obtain 100 vials each containing 100mg of the active ingredient.

N-[o-(p-pivaloyloxybenzenesulfonylamino)benzoyl]glycine

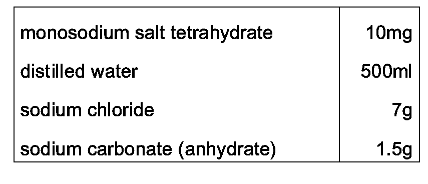

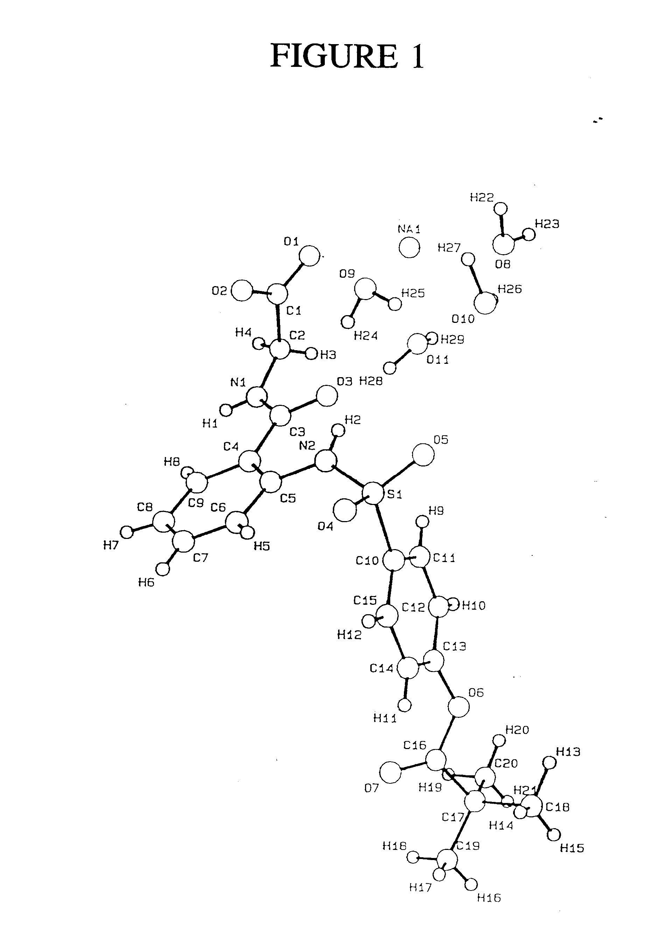

## Claims

1)N-[o-(p-pivaloyloxybenzenesulfonylamino)benzoyl]glycinemonosodiumsalttetrahydrateoftheformula (I):

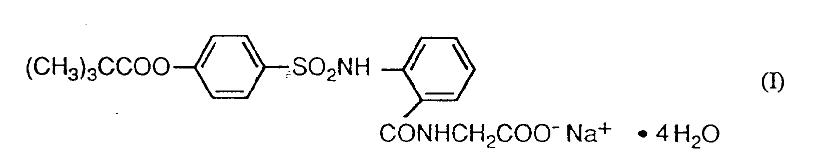

2) A process for the preparation of the compound of formula (I) claimed in claim 1, which comprises reacting a compound of the formula (II): 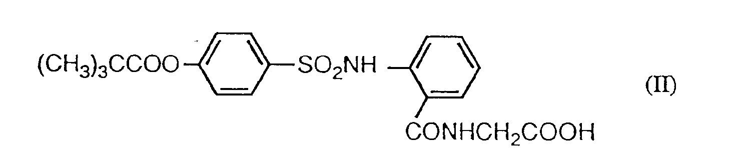
with an aqueous solution of sodium hydroxide, sodium bicarbonate or sodium carbonate in an inert organic solvent, recrystallizing the product obtained from water or a mixture of a water miscible organic solvent and water,
and then air-drying the crystals obtained.

3) A pharmaceutical composition which comprises the compound of the formula (I) claimed in claim 1, in association with a pharmaceutically acceptable diluent or carrier.

4)Acompound of the formula (I) as claimed in claim 1 for use in the prevention and/ortreatment of diseases induced by the abnormal enhancement of the degradation of elastin, collagen fiber and/or proteoglican by the action of elastase.

5) A process for the preparation of N-[o-(p-pivaloyloxybenzenesufonylamino)benzoyl]glycine of the formula (II) depicted in claim 2 as an intermediate for the preparation of a compound of the formula (I) claimed in claim 1, which process comprises reducing a compound of the formula (III):
